(19) Europäisches Patentamt
European Patent Office
Office européen des brevets

(11) Veröffentlichungsnummer: **0 004 938**
**B1**

(12) # EUROPÄISCHE PATENTSCHRIFT

(45) Veröffentlichungstag der Patentschrift: **03.06.81**

(51) Int. Cl.³: **C 07 D 499/12**, C 07 D 501/06

(21) Anmeldenummer: **79101110.9**

(22) Anmeldetag: **11.04.79**

(54) **Verfahren zur Herstellung halbsynthetischer beta-Lactamantibiotika.**

(30) Priorität: **20.04.78 DE 2817228**

(43) Veröffentlichungstag der Anmeldung:
**31.10.79 Patentblatt 79/22**

(45) Bekanntmachung des Hinweises auf die Patenterteilung:
**03.06.81 Patentblatt 81/22**

(84) Benannte Vertragsstaaten:
**BE CH DE FR GB IT NL SE**

(56) Entgegenhaltungen:
**DE-A-2 519 400**
**DE-A-2 613 172**

(73) Patentinhaber: **BAYER AG, Zentralbereich Patente, Marken und Lizenzen, D-5090 Leverkusen 1, Bayerwerk (DE)**

(72) Erfinder: **Ertel, Werner, Dr., Theodor-Heuss-Strasse 50, D-5600 Wuppertal 1 (DE)**

EP 0 004 938 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

# Verfahren zur Herstellung halbsynthetischer β-Lactamantibiotika

Die Anmeldung betrifft ein Verfahren zur Herstellung von halbsynthetischen β-Lactamantibiotika aus Aminoverbindungen der Formel

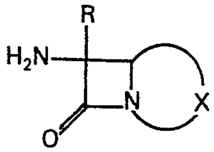

(I)

worin
X die restlichen Glieder eines in β-Lactamantibiotika üblichen Ringes und
R Wasserstoff oder Methoxy bedeuten,
und aktivierten Carbonsäurederivaten der Formel

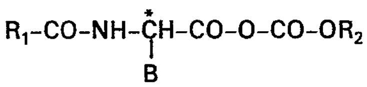

(II)

worin
$R_1$ für einen organischen Rest,
B für gegebenenfalls substituiertes Phenyl, Cyclohexadienyl oder Heterocyclyl und
$R_2$ für einen niederen Alkylrest stehen.

Es ist beispielsweise aus der DE-OS 25 19 400 bekannt, Verbindungen der Formel (I) mit Verbindungen der Formel (II) in wasserfreiem Aceton zu halbsynthetischen β-Lactamantibiotika umzusetzen. Dabei erhält man jedoch aus ursprünglich hinsichtlich des durch * gekennzeichneten Chiralitätszentrums optisch reinen Verbindungen der Formel (II) Endprodukte, die am Chiralitätszentrum eine teilweise Racemisierung erfahren haben.

Auch aus der DE-OS 26 13 172 ist das Arbeiten in Aceton/Wasser bekannt. Im Gegensatz zu dem dort beschriebenen Verfahren wird aber beim erfindungsgemässen Verfahren die Säure direkt aus dem Lösungsmittelgemisch durch Ansäuern gefällt. Während nach dem Verfahren der DE-OS das Aceton zunächst bei niedriger Temperatur und erniedrigtem Druck entfernt wird, die verbleibende Lösung mit Methylisobutylketon überschichtet wird und danach in mehreren Schritten das Reaktionsprodukt gewonnen wird. Diese Druckschrift gibt aber keinerlei Hinweise darauf, dass durch einfaches Ansäuern der Reaktionsmischung ein Produkt in reiner Form kristallin erhalten werden kann.

Ein weiterer und wesentlicher Vorteil des erfindungsgemässen Verfahrens liegt darin, dass optisch einheitliche Produkte erhalten werden, was keineswegs selbstverständlich ist. Vgl. hierzu auch: Houben-Weyl, Bd. 15 1, S. 34ff (Grundlagen der Peptid-Synthese; 22.40. Die α-Chiralität).

Da aus pharmakologischen Gründen optisch reine Endprodukte jedoch wesentlich wertvoller sind, weil sie eine höhere Wirkung und geringere Nebenwirkungen zeigen, bestand ein Bedürfnis, das oben geschilderte Verfahren so zu variieren, dass eine teilweise Racemisierung am Chiralitätszentrum nicht eintritt.

Es wurde nun überraschenderweise ein verbessertes Verfahren gefunden, das Produkte liefert, die die o.g. Nachteile nicht mehr aufweisen. Das Verfahren ist dadurch gekennzeichnet, dass man die Verbindungen (I) und (II) in Aceton-Wasser-Gemisch umsetzt, das Aceton und Wasser im Volumen-Verhältnis 0,5:1 bis 3:1 enthält, und anschliessend durch Ansäuern mit einer Mineralsäure das Umsetzungsprodukt ausfällt.

Das Verfahren ist insbesondere zur Herstellung optisch reiner Verbindungen der Formel

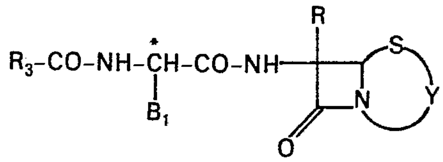

(III)

geeignet, worin
R Wasserstoff oder Methoxy,
$R_3$ einen Rest der Formel

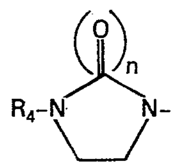

$R_4$ Wasserstoff, Alkyl, Aryl, Hetaryl, Cycloalkyl, Alkylsulfonyl, Arylsulfonyl, Hetarylsulfonyl, Cycloalkylsulfonyl, Alkylmethylimino, Arylmethylimino, Hetarylmethylimino oder Cycloalkylmethylimino,
$B_1$ Phenyl, 4-Hydroxyphenyl, 2-Furyl oder 2-Amino-1,3-thiadiazol-4-yl,
n 1 oder 2,
Y die Gruppen

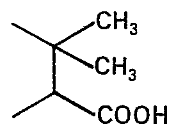

oder

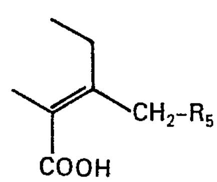

$R_5$ Wasserstoff, Acetoxy,

$-OCO-NR_6R_7$ oder

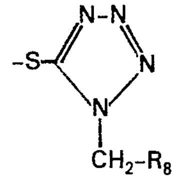

$R_6$ und $R_7$ Wasserstoff, Alkyl oder Cycloalkyl,
$R_8$ Wasserstoff, Carboxy, Sulfo oder $CH_2-NR_9R_{10}$ und
$R_9$ und $R_{10}$ Wasserstoff oder Alkyl bedeuten.

Vorzugsweise werden nach dem erfindungsgemässen Verfahren Verbindungen hergestellt, in denen

$R_4$ Wasserstoff, Methylsulfonyl, Cyclopropyl oder (Furyl-2)-Methylimino,

$R_5$ Wasserstoff, Acetoxy oder einen Rest der Formel

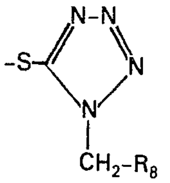

und

$R_8$ Wasserstoff oder Carboxy bedeuten und

R, $B_1$, n und Y die vorstehend genannte Bedeutung haben.

Das erfindungsgemässe Verfahren zeichnet sich gegenüber dem vorstehend beschriebenen Verfahren des Standes der Technik und gegenüber gleichfalls vorbekannten Verfahren, bei denen Verbindungen der Formel

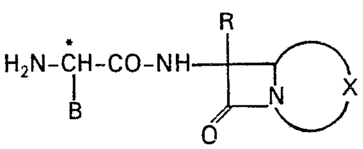 (IV)

worin

B, R und X die obengenannte Bedeutung haben, mit aktivierten Carbonsäuren der Formel

$R_1$-COOH

worin

$R_1$ die o.g. Bedeutung hat

umgesetzt werden, durch Verkürzung des Reaktionsweges und geringeren Aufwand aus.

Die für das erfindungsgemässe Verfahren erforderlichen Zwischenprodukte sind bekannt oder auf üblichem Wege zugänglich.

Sofern B bzw. $B_1$ die Bedeutung Hydroxyphenyl hat, wird jedoch zweckmässigerweise die entsprechende Verbindung der Formel II durch Silylierung von (4-Hydroxyphenyl)-glycin, Umsetzung mit $R_1$-COCl und Wasser hergestellt.

Das Verfahren wird zweckmässigerweise so durchgeführt, dass man die Verbindungen der Formel (I) und die Verbindungen der Formel (II) im Aceton-Wasser-Gemisch bei Temperaturen von -60°C bis +30°C, vorzugsweise -10°C bis +10°C in Gegenwart einer organischen Base bei Normaldruck miteinander umsetzt. Auf ein Mol der Verbindung der Formel (II) setzt man im allgemeinen 1 bis 1,5, vorzugsweise 1,1 bis 1,2 Mol der Verbindung der Formel (I) ein.

Als organische Basen kommen insbesondere sekundäre und tertiäre Amine wie Dimethylamin, Triäthylamin, N-Methylmorpholin oder Aminoalkohole in Frage. Vorzugsweise verwendet man 3-Dimethylaminopropanol. Pro Mol der Verbindung (II) setzt man 0,001 bis 0,01 Mol der organischen Base ein.

Das erfindungsgemässe Verfahren wird unter intensivem Durchmischen durchgeführt und ist etwa nach 20 bis 60 Minuten beendet.

Zur Aufarbeitung wird rasch mit Mineralsäure, beispielsweise mit Salzsäure, angesäuert, worauf das gewünschte Produkt in sehr reiner Form auskristallisiert. Die Reinheit des Produktes kann durch die verschiedenen Analysenmethoden wie NMR-Spektroskopie, HPL-Chromatographie, mikrobiologische Wirksamkeit, Drehwertbestimmung und Circulardichroismus bestimmt werden.

Das erfindungsgemässe Verfahren kann im selben Reaktionsgefäss durchgeführt werden, in dem die als Vorstufe erforderliche Verbindung (II) hergestellt wird.

Beispiel 1

D-α-[(2-oxo-1-imidazolinyl)-carbonyl-amino]-phenylessigsäure

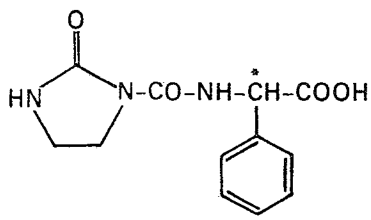 (1)

136 g D-Phenylglycin (2) werden in 2 l Wasser bei Raumtemperatur mit 450 ml 2 N NaOH bei pH 12 in Lösung gebracht. Anschliessend wird mit 450 ml 2 N HCl unter Kühlung auf pH 7,6 gestellt. (2) fällt fein verteilt wieder aus. Die Suspension wird mit 132 g 1-Chlorcarbonyl-imidazolidinon (3) versetzt und mit 810 ml 2 N NaOH unter Kühlen auf 20°C auf pH 7,7 eingestellt. Nach 15 Minuten hat sich das Säurechlorid weitgehend umgesetzt. Die Mischung wird filtriert, das Filtrat mit 2 N HCl auf pH 2 gebracht. Es fällt ein farbloser Niederschlag aus. Man rührt 20 Minuten nach, saugt ab und wäscht 2mal mit 100 ml Wasser. Der Filterrückstand wird bei Raumtemperatur im Vakuum getrocknet. Man erhält 176 g (75% der Theorie) der Verbindung (1) mit einem Gehalt von 98%.

NMR: $d_6$-DMSO; δ-Skala
(δ; Anzahl der Protonen; Art des Signals, Position der Protonen; Kopplungskonstante I [Hz])
3.31, 2, t, 4-$CH_2$;
3.7, 2, t, 5-$CH_2$, $J_{4-5}$ = 8,0;
5.29, 1, d, α-H, $I_{\alpha-N'}$ = 8,0;
7.33, 5, S(b), Phenyl;
7.58, 1, S, NH (Imidazolidinon);
9.05, 1, d, NH.

Beispiel 2

D-α-[2-oxo-1-imidazolidinyl)-carbonyl-amino]-benzylpenicillin

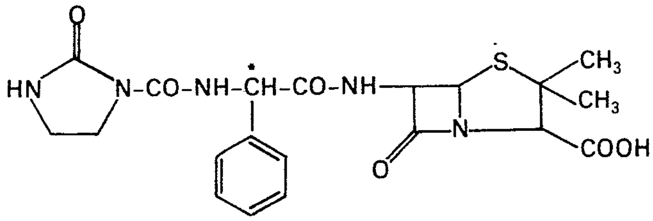

(4)

14,5 g (1) werden in 112 ml wasserfreiem Aceton mit 7,6 ml Triäthylamin versetzt. Die Innentemperatur der Mischung wird zwischen 20 bis 25 °C gehalten. Nach 10 Minuten hat sich ein weisser Niederschlag gebildet. Die Mischung wird auf -40 °C gekühlt und mit 0,13 ml 3-Dimethylaminopropanol-1 und 5,5 ml Chlorameisensäureäthylester versetzt. Die Temperatur der Mischung steigt auf -30 °C an. Bei dieser Temperatur wird 10 Minuten nachgerührt und anschliessend wieder auf -50 °C gekühlt. Es entsteht die Verbindung der Formel

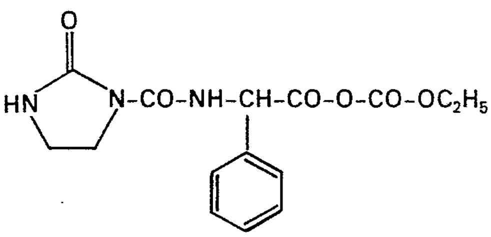

(5)

13,1 g 6-APS werden in 38 ml Wasser bei 0 bis 5 °C mit ca. 30 ml 2 N NaOH bei pH 7,8 innerhalb von ca. 20 Minuten in Lösung gebracht. Die wässrige Lösung wird zu 53 ml auf -20 °C gekühltes Aceton getropft. Die Mischung wird 5 Minuten gerührt und dabei auf -10 °C eingestellt. Diese Lösung wird in einem Schwung zur acetonischen Lösung von (5) gegeben. Man lässt die Innentemperatur auf 0 °C ansteigen. Es entsteht eine blanke Lösung. Nach Versetzen mit 200 ml Wasser wird das Aceton im Vakuum abgedampft, die zurückbleibende Lösung filtriert und mit ca. 70 ml 1 N HCl auf pH 2 eingestellt. Es fällt die Säure (4) aus. Der farblose Niederschlag wird abgesaugt, 2mal mit je 50 ml Wasser gewaschen und über $P_2O_5$ im Vakuum getrocknet. Man erhält 23 g (91% der Theorie) mit einem Gehalt von 95,5%. $[\alpha_D] = 201°$ (1%ige Lsg. in DMF), 187° (1%ige Lösung in Phosphat-Puffer bei pH 8).

Anstelle des aus (1) erhaltenen Triäthylenammoniumsalzes kann auch das entsprechende Natriumsalz hergestellt und verwendet werden, welches man durch Lösen von (1) mit 1 N NaOH bei pH 7,5 und anschliessendem Lyophilisieren erhält.

Beispiel 3

D-2-[3-(Methylsulfonyl)-2-oxo-imidazolidin-1-carboxamido]-2-phenylessigsaures Natriumsalz

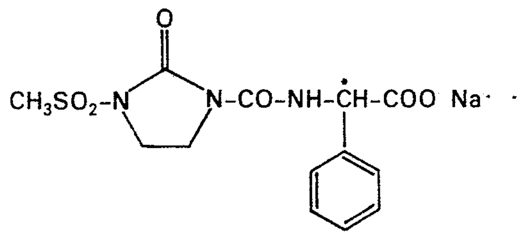

(6)

37,1 g (2) werden in 545 ml Wasser bei pH 12 durch Zugabe von 125 ml 2 N NaOH gelöst. Aus der Lösung wird durch Einstellen von pH 7,6 mit 120 ml 2 N HCl feinverteiltes wieder (2) ausgefällt. Die auf 20 °C gekühlte Suspension wird mit 60,7 g der Verbindung der Formel

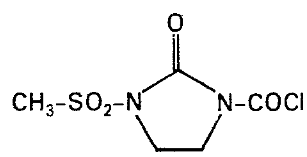

(7)

versetzt. Im Verlauf von ca. 1 Stunde werden 460 ml 1 N NaOH so zugegeben, dass ein pH von 7,6 nicht überschritten wird. Am Ende ist die Lösung fast blank. Geringe Feststoffanteile werden durch Filtrieren entfernt. Aus dem Filtrat wird durch Ansäuern mit ca. 240 ml 1 N HCl auf pH 2 die freie Säure des Salzes (6) als farbloser Niederschlag ausgefällt. Der Niederschlag wird abfiltriert, 2mal mit 100 ml Wasser gewaschen und anschliessend zum Na-Salz durch Aufschlämmen in 100 ml Wasser, gleichzeitiges Zutropfen von 2 N NaOH bei pH 7,6, Abfiltrieren und anschliessendes Lyophilisieren aufgearbeitet. Man erhält 49 g (55% der Theorie) mit einem Gehalt von 94%.

Beispiel 4

6-[D-2[3-(Methylsulfonyl)-2-oxo-imidazolidin-1-carboxamido]-2-phenyl-acetamido]-penicillansäure

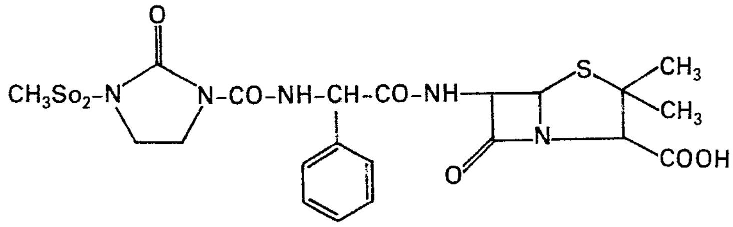

(8)

112 ml wasserfreies Aceton werden auf -40°C gekühlt und nacheinander mit 5,5 ml Chlorameisensäureäthylester, 0,13 ml 3-Dimethylaminopropanol-1 und 21 g (6) versetzt. Nach Entfernen des Kühlbades steigt die Innentemperatur auf -30°C an. Nach 10minütigem Nachrühren bei -30°C wird wieder auf -50°C abgekühlt. Die Mischung wird sodann mit einer wässrig-acetonischen Lösung von 6-APS-Natrium versetzt. Hergestellt durch Auflösen von 13,1 g 6-APS in 37 ml auf 0°C gekühltem Wasser bei pH 7,8 durch Eintropfen von 30 ml 2 N NaOH und Zugabe dieser Lösung zu 53 ml auf -20°C gekühltem Aceton mit anschliessendem Nachrühren bei -15°C bis -10°C, bis eine blanke Lösung entstanden ist.

Nach Zugabe des 6-APS-Na-Lösung lässt man die Innentemperatur der Mischung auf 0°C ansteigen, versetzt mit 200 ml Wasser und dampft das Aceton im Vakuum bei 20 bis 30°C ab. Die zurückbleibende trübe Lösung wird mit einigen Tropfen Natronlauge auf pH 7 gebracht, über Kieselgur filtriert und mit ca. 70 ml 1 N HCl auf pH 2 eingestellt. Der farblose Niederschlag wird abfiltriert, 2mal mit je 100 ml Wasser gewaschen und im Vakuum bei 20°C über $P_2O_5$ getrocknet. Man erhält 25,2 g (85% der Theorie) mit einem Gehalt von 90%. $[\alpha_D] = +168°$ (1%ig in Phosphat-Puffer bei pH 8).

Anstelle des Na-Salzes (6) kann auch das Triäthylammoniumsalz eingesetzt werden, welches analog Beispiel 2 in acetonischer Lösung hergestellt wird.

Beispiel 5

D-α-[3-Furfurylidenamino-2-oxo-imidazolidin-1-carboxamido]-phenylessigsäure

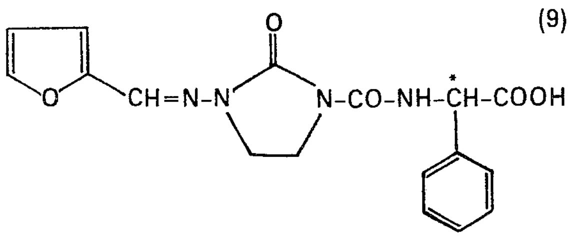

(9)

27,2 g (2) werden in 400 ml Wasser durch Zugabe von ca. 95 ml 2 N NaOH in Lösung gebracht bis ein pH von 12 erreicht ist. Mit 15 ml 2 N HCl wird (2) wieder in feinverteilter Form bei pH 7,6 ausgefällt. Die Suspension wird mit 44 g der Verbindung der Formel

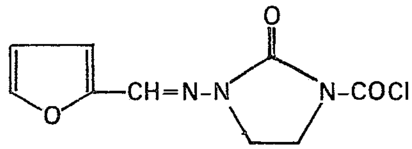

(10)

versetzt. Durch Zutropfen von ca. 360 ml 1 N NaOH bei einem pH-Wert zwischen 7,5 und 7,7 entsteht im Verlauf von ca. 1,5 Stunden allmählich eine blanke Lösung. Man saugt von Feststoffresten ab und dosiert zum Filtrat ca. 195 ml 1 N HCl zu, bis pH 2 erreicht ist. Der dabei ausfallende Niederschlag wird abgesaugt, 2mal mit je 200 ml Wasser gewaschen und im Vakuum bei 20°C über $P_2O_5$ getrocknet. Man erhält 61,5 g (96% der Theorie) mit einem Gehalt von 96,4%.

NMR: 3.77, 4, s(b), $CH_2$-$CH_2$;
5.35, 1, d, α-H, $I_{\alpha-N} = 7{,}5$;
6.52, 1, dd, 4-H (Furan), $I_{4-5} = 1{,}6$;
6.78, 1, d, 3-H (Furan), $I_{3-4} = 3{,}6$;
7.36, 5, s(b), Phenyl;
7.68, 1, s, Azomethin-H;
7.76, 1, d, 5-H (Furan).

Beispiel 6

6-{D-[3-Furfurylidenamino-2-oxo-imidazolidin-1-carboxamido]-phenylacetamido}-penicillansäure

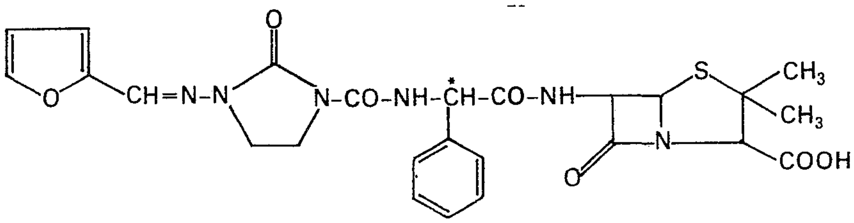

(11)

19,6 g (9) werden in 280 ml wasserfreiem Aceton mit 7,6 ml Triäthylamin versetzt, wobei die Innentemperatur zwischen 20 und 25°C gehalten wird. Nach etwa 2 Minuten entsteht eine blanke Lösung, die nach weiteren 10 Minuten Nachrühren auf -40°C abgekühlt wird. Bei ca. 0°C fällt das Triäthylammoniumsalz als voluminöser Niederschlag aus. Die Mischung wird mit 0,13 ml 3-Dimethylaminopropanol-1 und 5,5 ml Chlorameisensäureäthylester versetzt. Es bildet sich das gemischte Anhydrid der Formel

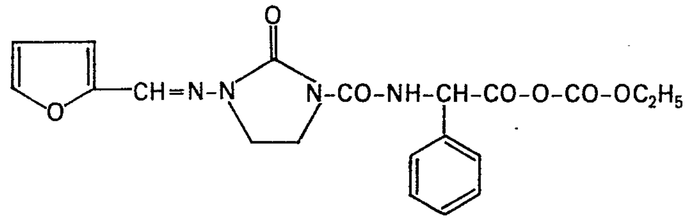

(12)

Die Innentemperatur steigt auf -30 °C an. Bei dieser Temperatur wird 10 Minuten nachgerührt, wobei die Mischung dünnflüssiger wird. Nach Abkühlen auf -50 °C wird eine wässrig-acetonische 6-APS-Na-Salz-Lösung auf einmal zugegeben, hergestellt durch Auflösen von 13,1 g 6-APS in 37 ml auf 0 °C gekühltem Wasser bei pH 7,5 bis 7,8 durch Eintropfen von 30 ml 2 N NaOH und Zugabe dieser Lösung zu 53 ml auf -20 °C gekühltem Aceton mit anschliessendem Nachrühren bei -15 bis -10 °C bis eine blanke Lösung entstanden ist.

Nach Vereinigen der beiden Lösungen lässt man die Temperatur allmählich auf 0 °C ansteigen, versetzt dann mit 200 ml Wasser und dampft das Aceton im Vakuum bei 20 °C ab. Man füllt die Lösung mit Wasser auf 750 ml auf, saugt von Feststoffresten ab und stellt das Filtrat mit ca. 53 ml 1 N HCl auf pH 2. Der farblose Niederschlag wird abgesaugt, 2mal mit je 50 ml gewaschen und im Vakuum bei 20 °C über $P_2O_5$ getrocknet. Man erhält 29 g (95% der Theorie) mit einem Gehalt von 92,5%. $[\alpha_D]$ = +222.6° (0,5%ige Lösung in Phosphat-Puffer bei pH 8).

Anstelle des Triäthylammoniumsalzes kann auch das entsprechende Na-Salz eingesetzt werden.

NMR: 1,47, 3, s, 2-$CH_3$;
3.85, 4, s(v), $CH_2$-$CH_2$;
3.99, 1, s, 3-H;
5.34, 1, d, 5-H, $I_{5-6}$ = 3,9;
5.47, 1, dd, 6-H, $I_{6-N}$ = 8,0;
5.78, 1, d, α-H, $I_{\alpha-N}$ = 8,0;
6.64, 1, dd, 4-H (Furan), $I_{4-5}$ = 1,6;
6,89, 1, d, 3-H (Furan), $I_{3-4}$ = 3,5;
7.2–7.5, 5, m, Phenyl;
7.78, 1,s, Azomethin-H;
7.84, 1, d, 5-H (Furan);
9,08, 1, d, 6-N-H;
9.15, 1, d, α-N-H.

Beispiel 7

D-α-[3-Furfuryliden-2-oxo-imidazolidin-1-carboxamido]-p-hydroxy-phenyl-essigsäure

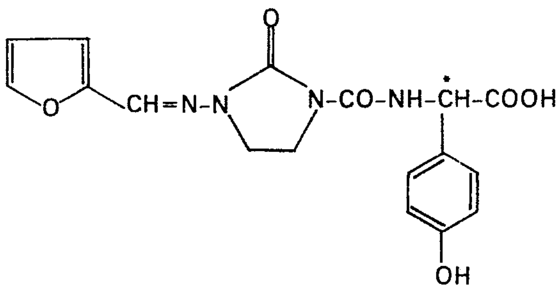

(13)

Zu 33,4 g D-p-Hydroxy-phenylglycin (14) und 91,2 g Triäthylamin werden in 420 ml $CH_2Cl_2$ bei Raumtemperatur unter Wasserkühlung (Innentemperatur: 20 bis 25 °C) innerhalb von 30 Minuten 76,8 g Trimethylchlorsilan (15) gelöst in 100 ml $CH_2Cl_2$, zugetropft.

Danach rührt man ohne zu heizen über Nacht nach. Es bildet sich eine hellbeige Suspension. Man destilliert bei Normaldruck ca. 360 ml $CH_2Cl_2$ ab, kühlt die Mischung auf 20 °C und saugt vom ausgefallenen Triäthylammoniumhydrochlorid ab. Der Filterrückstand wird 2mal mit je 80 $CH_2Cl_2$ gewaschen und die Waschlauge mit dem Filtrat vereinigt. Unter Kühlung werden in das Filtrat portionsweise innerhalb von 30 Minuten 48,5 g (10) eingetragen. Die Innentemperatur beträgt 20 bis 25 °C. Am Ende liegt eine braun-schwarze Lösung vor. Man rührt 15 Minuten nach und dampft dann im Vakuum bei 20 °C zur Trockne ein.

Der Rückstand wird in 400 ml wasserfreiem Aceton gelöst, die Lösung 5 Minuten mit 15 g A-Kohle verrührt und über ein AS-Filter abgesaugt.

Man lässt die klare braune Lösung unter kräftigem Rühren in 600 ml Wasser einlaufen, rührt 10 Minuten nach, währenddessen sich ein anfangs amorpher, dann zunehmend kristalliner farbloser Niederschlag bildet und saugt ab. Der Filterrückstand wird 2mal mit je 100 ml Wasser, dann 2mal mit je 50 ml Aceton nachgewaschen und im Vakuum bei 20 °C über $P_2O_5$ getrocknet. Man erhält 68,5 g (92% der Theorie) mit einem Gehalt von 94,0%.

NMR: 3.77, 4, s(b), $CH_2$-$CH_2$;
5.19, 1, d, α-H, $I_{\alpha-N}$ = 7,5;
6.55, 1, dd, 4-H (Furan), $I_{4-5}$ = 1,6;
6.77, 2, d, 2,2′-H (Phenyl), $I_{2-3}$ = 8,5;
6.82, 1, d, 3-H (Furan), $I_{3-4}$ = 3,5;
7.1, 2, d, 3,3′-H (Phenyl);
7.7, 1, s, Azomethin-H;
7.77, 1, d, 5-H (Furan);
8.95, 1, d, N-H;
9.5, 1, s(b), COOH.

Beispiel 8

6-[D-α-[3-Furfurylidenamino-2-oxo-imidazolidin-1-carboxamido]-p-hydroxy-phenyl-acetamido-penicillansäure

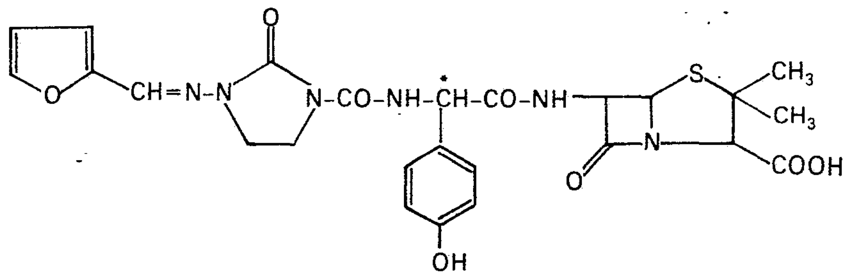

(14)

20,4 g (13) werden in 280 ml wasserfreiem Aceton unter Feuchtigkeitsausschluss mit 7,6 ml Triäthylamin versetzt. Nach ca. 3 Minuten beginnt die Umwandlung ins Triäthylammoniumsalz, erkennbar an der Bildung eines feinteiligen voluminösen Niederschlags.

Nach 10 Minuten Nachrühren wird auf -10°C gekühlt. Die Mischung wird sodann mit 0,13 ml 3-Dimethylaminopropanol und 5,5 ml Chlorameisensäureäthylester versetzt. Die Temperatur steigt dabei auf 0°C an. Im Verlaufe von 45 Minuten wird die Mischung dabei allmählich dünnflüssiger. Es entsteht das gemischte Anhydrid der Formel

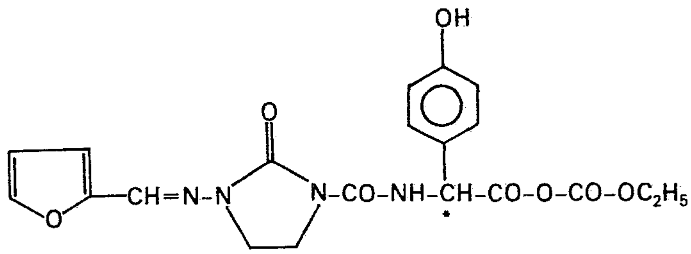

Danach kühlt man wieder auf -10°C und versetzt mit einer wässrig-acetonischen 6-APS-Na-Salz-Lösung, hergestellt durch Auflösen von 13.1 g 6-APS in 37 ml auf 0°C gekühltem Wasser bis pH 7,5-7,8 durch Eintropfen von 30 ml 2 N NaOH und Zugabe dieser Lösung zu 53 ml auf -20°C gekühltem Aceton mit anschliessendem Nachrühren bei -15°C bis -10°C, bis eine blanke Lösung entstanden ist. Nach Vereinigen der Mischungen hielt man die Temperatur auf -10°C und rührt 1 Stunde nach. Dann lässt man die Innentemperatur auf -5°C ansteigen und säuert rasch mit ca. 70 ml 1 N HCl auf pH 2 an. Die Mischung wird dabei kurzzeitig blank. Einige Minuten nach Erreichen des End-pH beginnt die kristalline Säure auszufallen. Man rührt 1 Stunde nach und saugt dann den farblosen Niederschlag ab. Der Filterkuchen wird 2× mit je 100 ml Wasser gewaschen und 30 Minuten trockengesaugt. Anschliessend trocknet man im Vakuum bei 20°C über Phosphorpentoxid bis zur Gewichtskonstanz.

Ausbeute: 25 g = 79% d. Theorie

Gehalt: 96%

$[\alpha_D]$ = +233° (1%ige Lösung in Phosphat-Puffer bei pH 8)

Anstelle des Triäthylammoniumsalzes kann auch das entsprechende Na-Salz eingesetzt werden.

NMR: 1.47, 3, s. 2-$CH_3$;
1.59, 3, s, 2-$CH_3$;
3.84, 4, s(b), $CH_2$-$CH_2$;
3.95, 1, s, 3-H;
5.35, 1, d, 5-H, $I_{5\text{-}6}$ = 3,9;
5.47, 1, dd, 6-H, $I_{6\text{-}N}$ = 8,0;
5.65, 1, d, α-H, $I_{\alpha\text{-}N}$ = 8,0;
6.64, 1, dd, 4-H (Furan), $I_{4\text{-}5}$ = 1,6;
6.77, 2, d, 2,2'-H (Phenyl), $I_{2\text{-}3}$ = 8,5;
6.87, 1, d, 3-H (Furan), $I_{3\text{-}4}$ = 3,6;
7.23, 2, d, 3,3'-H (Phenyl);
7.7, 1, s, Azomethin-H;
7.84, 1, d, 5-H (Furan);
8.96, 1, d, 6-NH;
8.99, 1, d, α-N-H.

**Patentansprüche**

1. Verfahren zur Herstellung von halbsynthetischen β-Lactamantibiotika aus Aminoverbindungen der Formel

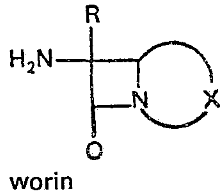

(I)

worin

X die restlichen Glieder eines in β-Lactamantibiotika üblichen Ringes und

R Wasserstoff oder Methoxy bedeuten, und aktivierten Carbonsäurederivaten der Formel

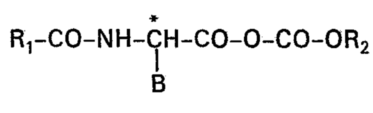

(II)

worin

$R_1$ für einen heterocyclischen organischen Rest,

B für gegebenenfalls durch Hydroxyl substituiertes Phenyl, Cyclohexadienyl oder Heterocyclyl und

$R_2$ für einen niederen Alkylrest stehen,

dadurch gekennzeichnet, dass man die Verbindungen (I) und (II) in Aceton-Wasser-Gemisch umsetzt, das Aceton und Wasser im Volumen-Verhältnis 0,5:1 bis 3:1 enthält, und anschliessend durch Ansäuern mit einer Mineralsäure das Umsetzungsprodukt ausfällt.

2. Verfahren nach Anspruch 1, dadurch gekennzeichnet, dass man Verbindungen der Formel

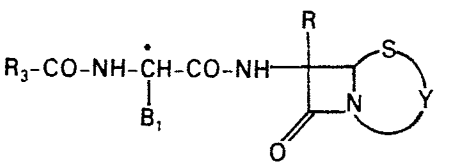
(III)

herstellt,

worin

R Wasserstoff oder Methoxy,

$R_3$ einen Rest der Formel

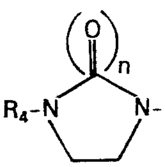

$R_4$ Wasserstoff, Alkyl, Aryl, Hetaryl, Cycloalkyl, Alkylsulfonyl, Arylsulfonyl, Hetarylsulfonyl, Cycloalkylsulfonyl, Alkylmethylimino, Arylmethylimino, Hetarylmethylimino oder Cycloalkylmethylimino,

$B_1$ Phenyl, 4-Hydroxyphenyl, 2-Furyl oder 2-Amino-1,3-thiadiazol-4-yl,

n 1 oder 2,

Y die Gruppen

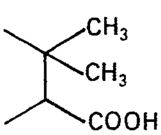
oder
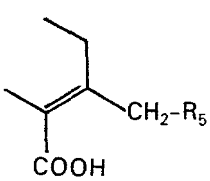

$R_5$ Wasserstoff, Acetoxy,

$-OCO-NR_6R_7$ oder 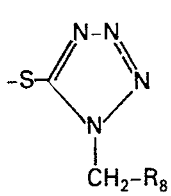

$R_6$ und $R_7$ Wasserstoff, Alkyl oder Cycloalkyl,

$R_8$ Wasserstoff, Carboxy, Sulfo oder $CH_2-NR_9R_{10}$

und

$R_9$ und $R_{10}$ Wasserstoff oder Alkyl bedeuten.

3. Verfahren nach Anspruch 2, dadurch gekennzeichnet, das man Verbindungen herstellt, in denen

$R_4$ Wasserstoff, Methylsulfonyl, Cyclopropyl oder (Furyl-2)-Methylimino,

$R_5$ Wasserstoff, Acetoxy oder einen Rest der Formel

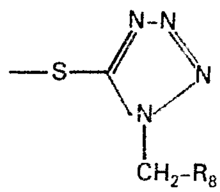

und

$R_8$ Wasserstoff oder Carboxy bedeuten und

R, $B_1$, n und Y die in Anspruch 1 genannte Bedeutung haben.

4. Verfahren nach Anspruch 1, dadurch gekennzeichnet, dass man die Umsetzung bei Temperaturen von -60 °C bis +30 °C durchführt.

5. Verfahren nach Anspruch 1, dadurch gekennzeichnet, dass man die Umsetzung bei Temperaturen von -10 °C bis +10 °C durchführt.

6. Verfahren nach Anspruch 1, dadurch gekennzeichnet, dass man die Umsetzung in Gegenwart einer organischen Base durchführt.

7. Verfahren nach Anspruch 1, dadurch gekennzeichnet, dass man auf 1 Mol der Verbindung der Formel II 1 bis 1,5 Mol der der Verbindung der Formel I einsetzt.

8. Verfahren nach Anspruch 1, dadurch gekennzeichnet, dass man auf 1 Mol der Verbindung der Formel II 1,1 bis 1,2 Mol der Verbindung der Formel I einsetzt.

## Claims

1. Process for the preparation of semi-synthetic β-lactam antibiotics from amino compounds of the formula

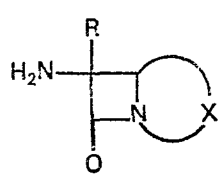
(I)

wherein

X denotes the remaining members of a ring customary in β-lactam antibiotics and

R denotes hydrogen or methoxy,

and activated carboxylic acid derivatives of the formula

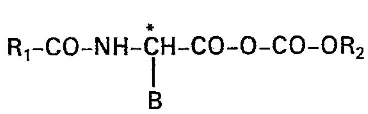
(II)

wherein

$R_1$ represents a heterocyclic organic radical,

B represents optionally hydroxyl-substituted phenyl, cyclohexadienyl or heterocyclyl and

$R_2$ represents a lower alkyl radical,

characterised in that the compounds (I) and (II) are reacted in an acetone/water mixture which contains acetone and water in a volume-ratio of 0.5:1 to 3:1, and then the reaction product is precipitated by acidification with a mineral acid.

2. Process according to Claim 1, characterised in that compounds of the formula

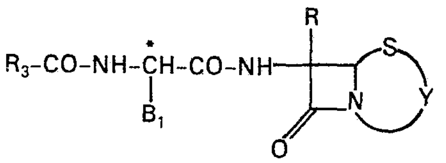

(III)

wherein

R denotes hydrogen or methoxy,

$R_3$ denotes a radical of the formula

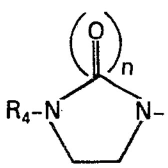

$R_4$ denotes hydrogen, alkyl, aryl, hetaryl, cycloalkyl, alkylsulphonyl, arylsulphonyl, hetarylsulphonyl, cycloalkylsulphonyl, alkylmethylimino, arylmethylimino, hetarylmethylimino or cycloalkylmethylimino,

$B_1$ denotes phenyl, 4-hydroxyphenyl, 2-furyl or 2-amino-1,3-thiadiazol-4-yl,

n denotes 1 or 2,

Y denotes the groups

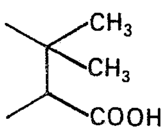

or

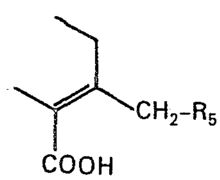

$R_5$ denotes hydrogen, acetoxy, $-OCO-NR_6R_7$ or

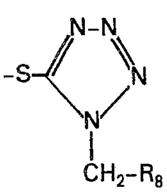

$R_6$ and $R_7$ denote hydrogen, alkyl or cycloalkyl, and

$R_9$ and $R_{10}$ denote hydrogen or alkyl,

are prepared.

3. Process according to Claim 2, characterised in that compounds

in which

$R_4$ denotes hydrogen, methylsulphonyl, cyclopropyl or (fur-2-yl)-methylimino,

$R_5$ denotes hydrogen, acetoxy or a radical of the formula

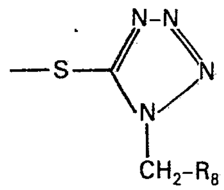

$R_3$ denotes hydrogen or carboxyl and

R, $B_1$,n and Y have the meaning given in Claim 1, are prepared.

4. Process according to Claim 1, characterised in that the reaction is carried out at temperatures from -60 °C to +30 °C.

5. Process according to Claim 1, characterised in that the reaction is carried out at temperatures from -10 °C to +10 °C.

6. Process according to Claim 1, characterised in that the reaction is carried out in the presence of an organic base.

7. Process according to Claim 1, characterised in that 1 to 1.5 mols of the compound of the formula I are employed per 1 mol of the coumpound of the formula II.

8. Process according to Claim 1, characterised in that 1.1 to 1.2 mols of the compound of the formula I are employed per 1 mol of the compound of the formula II.

**Revendications**

1. Procédé de préparation d'antibiotiques semi-synthétiques du type β-lactame à partir de composés aminés de formule:

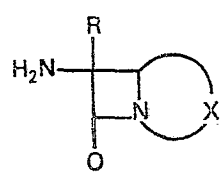

(I);

dans laquelle:

X représente les chaînons restants d'un cycle usuel dans les antibiotiques du type β-lactame, et

R représente l'hydrogène ou un groupe méthoxy, et de dérivés activés d'acides carboxyliques de formule:

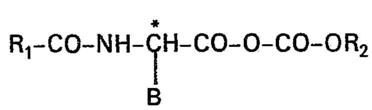

(II)

dans laquelle:

$R_1$ représente un reste organique hétérocyclique,

B représente un groupe phényle, cyclohexa-

diényle ou un reste hétérocyclique éventuellement substitué par des groupes hydroxy, et

$R_2$ représente un groupe alkyle inférieur, caractérisé en ce que l'on fait réagir les composés (I) et (II) dans un mélange acétone-eau contenant l'acétone et l'eau dans des proportions en volume de 0,5:1 à 3:1, et on précipite ensuite le produit de réaction par acidification à l'aide d'un acide minéral.

2. Procédé selon la revendication 1, caractérisé en ce que l'on prépare des composés de formule:

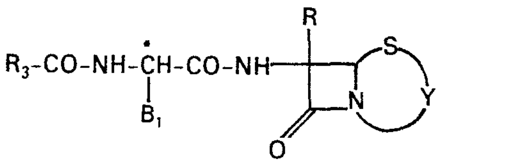

(III)

dans laquelle:

R représente l'hydrogène ou le groupe méthoxy,

$R_3$ représente un reste de formule:

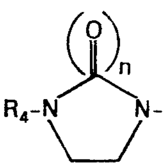

$R_4$ représente l'hydrogène, un groupe alkyle, aryle, hétéroaryle, cycloalkyle, alkylsulfonyle, arylsulfonyle, hétéroarylsulfonyle, cycloalkylsulfonyle, alkylméthylimino, arylméthylimino, hétéroarylméthylimino ou cycloalkylméthylimino,

$B_1$ représente un groupe phényle, 4-hydroxyphényle, 2-furyle ou 2-amino-1,3-thiadiazole-4-yle,

n est égal à 1 ou 2,

Y représente les groupes:

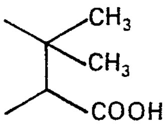

ou

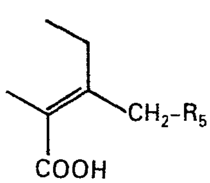

$R_5$ représente l'hydrogène, un groupe acétoxy

$-OCO-NR_6R_7$ ou 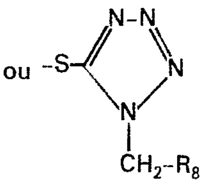

$R_6$ et $R_7$ représentent des atomes d'hydrogène, des groupes alkyle ou cycloalkyle,

$R_8$ représente l'hydrogène, un groupe carboxy, sulfo ou $CH_2-NR_9R_{10}$, et

$R_9$ et $R_{10}$ représentent des atomes d'hydrogène ou des groupes alkyle.

3. Procédé selon la revendication 2, caractérisé en ce que l'on prépare des composés dans lesquels:

$R_4$ représente l'hydrogène, un groupe méthylsulfonyle, cyclopropyle ou (furyl-2)-méthylimino,

$R_5$ représente l'hydrogène, un groupe acétoxy ou un reste de formule:

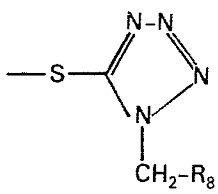

et $R_8$ représente l'hydrogène ou un groupe carboxy, et

R, $B_1$, n et Y ont les significations indiquées dans la revendication 1.

4. Procédé selon la revendication 1, caractérisé en ce que l'on effectue la réaction à des températures de -60 à +30 °C.

5. Procédé selon la revendication 1, caractérisé en ce que l'on effectue la réaction à des températures de -10 à +10 °C.

6. Procédé selon la revendication 1, caractérisé en ce que l'on effectue la réaction en présence d'une base organique.

7. Procédé selon la revendication 1, caractérisé en ce que, pour 1 mole du composé de formule (II), on utilise 1 à 1,5 mole du composé de formule (I).

8. Procédé selon la revendication 1, caractérisé en ce que, pour 1 mole du composé de formule (II), on utilise 1,1 à 1,2 mole du composé de formule (I).